(19) Europäisches Patentamt
European Patent Office
Office européen des brevets

(11) Publication number : **0 511 152 A2**

(12) # EUROPEAN PATENT APPLICATION

(21) Application number : **92610024.9**

(22) Date of filing : **06.04.92**

(51) Int. Cl.[5] : **C07D 241/38,** A61K 31/495, C07D 403/12

(30) Priority : **22.04.91 DK 730/91**

(43) Date of publication of application : **28.10.92 Bulletin 92/44**

(84) Designated Contracting States : **GB**

(71) Applicant : **NOVO NORDISK A/S**
**Novo Allé**
**DK-2880 Bagsvaerd (DK)**

(72) Inventor : **Jakobsen, Poul**
**Padborgvej 31**
**DK-2610 Rodovre (DK)**
Inventor : **Nielsen, Flemming Elmelund**
**Fureso Parkvej 46**
**DK-2830 Virum (DK)**
Inventor : **Naerum, Lars**
**Alrunevej 14**
**DK-2900 Hellerup (DK)**

(74) Representative : **Madsen, Inger Margrethe Schelde et al**
**c/o Novo Nordisk A/S, Patent Department,**
**Novo Allé**
**DK-2880 Bagsvaerd (DK)**

(54) **Quinoxaline compounds, their preparation and use.**

(57) Quinoxaline compounds having the formula I

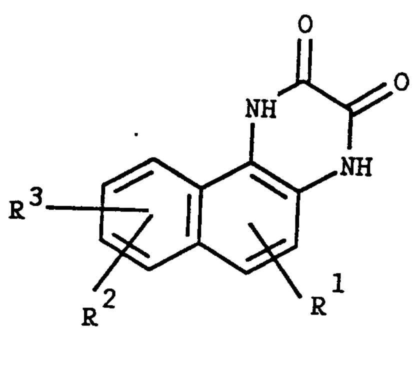

(I)

wherein

$R^1$ is H, $NO_2$, CN, $CF_3$ or halogen, $R^2$ and $R^3$ independently are H, CN, $CF_3$, halogen, $C(NOH)C_{1-6}$-alkyl, $COR^4$ or $SO_2R^4$, wherein $R^4$ is $C_{1-6}$-alkyl, optionally substituted, or $NR^5R^6$, wherein $R^5$, $R^6$ independently are H, $C_{3-6}$-cycloalkyl, $C_{1-6}$-alkyl-, optionally substituted, compositions thereof and methods of preparing the compounds are described.

The compounds are useful in the treatment of indications caused by hyperactivity of the excitatory neurotransmitters.

EP 0 511 152 A2

The present invention relates to therapeutically active heterocyclic compounds, a method of preparing the same, pharmaceutical compositions comprising the compounds, and a method of treating therewith.

L-glutamic acid, L-aspartic acid and a number of other closely related amino acids have in common the ability to activate neurons in the central nervous system (CNS). Bio-chemical, electrophysiological and pharmacological studies have substantiated this and demonstrated that acidic amino acids are transmitters for the vast majority of excitatory neurons in the mammalian CNS.

Interaction with glutamic acid mediated neurotransmission is considered a useful approach in the treatment of neurological and psychiatric diseases. Thus, known antagonists of excitatory amino acids have shown potent anxiolytic (Stephens et al., Psychopharmacology 90, 143-147, 1985), anticonvulsant (Croucher et al., Science 216, 899-901, 1982) and muscle relaxant properties (Turski et al., Neuro-sci. Lett. 53, 321-326, 1985).

It has been suggested that accumulation of extracellular excitatory amino acids, followed by overstimulation of neurons, may explain the neuronal degenerations seen in neurological disorders such as amyotrophic lateral sclerosis, Parkinsonism, Alzheimer's disease, Huntington's disease, epilepsy, and deficiencies of mental and motor performance seen after conditions of brain ischemia, anoxia and hypoglycemia or head and spinal cord trauma (McGeer et al., Nature 263, 517-519, 1976; Simon et al., Science 226, 850-852, 1984; Wieloch, Science 230, 681-683, 1985; Faden et al., Science 244, 798-800, 1989; Turski et al., Nature 349, 414-418, 1991). Other possible indications are psychosis, muscle rigidity, emesis and analgesia.

Excitatory amino acids exert their actions via specific receptors located postsynaptically or presynaptically. Such receptors are at present conveniently subdivided into three groups based on electrophysiological and neurochemical evidence: 1 the NMDA (N-methyl-D-aspartate) receptors, 2 the AMPA receptors,and 3 the kainate receptors. L-glutamic acid and L-aspartic acid probably activate all the above types of excitatory amino acid receptors and possibly other types as well.

The above mentioned classification of excitatory amino acid receptors into NMDA, AMPA, and kainate receptors is based primarily on the following electrophysiological and neurochemical findings.

1) N-methyl-D-aspartate (NMDA) receptors exhibit high selectivity for the excitant NMDA. Ibotenic acid, L-homocysteic acid, D-glutamic acid and trans-2,3-piperidine dicarboxylic acid (trans-2,3-PDA) exert a strong to moderate agonist activity on these receptors. The most potent and selective antagonists are the D-isomers of the 2-amino-5-phosphonocarboxylic acids, e.g. 2-amino-5-phosphono-valeric acid (D-APV) and 3-[(±)-2-carboxypiperazin-4-yl]-propyl-1-phosphonic acid (CPP), while moderate antagonist activity is shown by the D-isomers of long chain 2-amino dicarboxylic acids (e.g. D-2-amino-adipic acid) and long chain diaminodicarboxylic acids (e.g. diaminopimelic acid). The NMDA-induced synaptical responses have been extensively investigated in the mammalian CNS, especially in the spinal cord (J. Davies et al., J. Physiol. 297, 621-635, 1979) and the responses have been shown to be strongly inhibited by $Mg^{2+}$.

2) AMPA receptors are activated selectively by AMPA (2-amino-3-hydroxy-5-methyl-4-isoxazolepropionic acid), other potent agonists being quisqualic acid and L-glutamic acid. Glutamic acid diethyl ester (GDEE) is a selective but very weak antagonist of this site. AMPA receptors are relatively insensitive to $Mg^{2+}$.

Glutamate release has long been thought to play a major role in neuronal death resulting from cerebral ischemia (Benveniste, H. et al., J. Neurochem. 43, 1369-1374, 1984). It is well known that NMDA receptor evoked $Ca^{2+}$ influx is an important mechanism in ischemic neuronal cell loss. The non-NMDA receptor coupled ionophor is not permeable to calcium. However, the excitation by the Scaffer collaterals in the CA1 region is excerted by non-NMDA receptors, and this fact is of importance for the events in the postischemic period. Recent studies have shown that selective AMPA antagonists have neuroprotectant effects in global ischemia in the gerbil even when given several hours after reperfusion (Sheardown et al., Science 247, 571-574, 1990).

AMPA antagonists are therefore useful in the treatment of cerebral ischemia.

3) Kainate receptors. Excitatory responses to kainic acid are relatively insensitive to antagonism by NMDA-antagonists and by GDEE, and it has been proposed that kainic acid activates a third subclass of acidic amino acid receptor. Certain lactonized derivatives of kainic acid are selective antagonists (O. Goldberg et al., Neurosci. Lett. 23, 187-191, 1981) and the dipeptide 3-glutamyl-glycine also shows some selectivity for kainate receptors. $Ca^{2+}$ but not $Mg^{2+}$ is a strong inhibitor of kainic acid binding.

The affinity of a substance for one or more of the different types of excitatory amino acid receptors may be studied in simple binding experiments. In essense, the method involves incubation of a particular selected radiolabelled ligand and the particular specific substance to be investigated with brain homogenate which contains the receptor. Measurement of receptor occupancy is made by determination of the radioactivity bound to the homogenate and subtraction of nonspecific binding.

AMPA receptor binding may be studied by using $^3$H-AMPA as radioligand.

The influence of glutamic acid analogues on secondary effects of glutamate receptor interactions may be studied in vitro by using the phenomenon of spreading depression in chicken retina. Such experiments will provide information as to the efficacies (agonist/antagonist) of the test substances. This is in contrast to binding studies, which only provide information on the affinities of the compounds for the receptor.

It has now been found that the quinoxaline compounds of the invention have affinity for the AMPA receptors and are antagonists in connection with this type of receptor which makes them useful in the treatment of any of the numerous indications caused by hyperactivity of excitatory amino acids.

The quinoxaline compounds of the invention have the general formula I

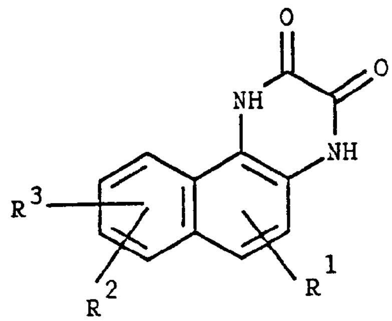

(I)

wherein

$R^1$ is hydrogen, $NO_2$, CN, $CF_3$ or halogen, $R^2$ and $R^3$ independently are hydrogen, CN, $CF_3$, halogen, $C(NOH)C_{1-6}$-alkyl, $COR^4$ or $SO_2R^4$, wherein $R^4$ is $C_{1-6}$-alkyl, optionally substituted with $CONH_2$, or $NR^5R^6$, wherein $R^5$, $R^6$ independently are hydrogen, $C_{3-6}$-cycloalkyl, $C_{1-6}$-alkyl optionally substituted with $CONH_2$ or 2-oxo-pyrrolidinyl, or pharmaceutically acceptable salts thereof.

The invention also relates to a method of preparing the above-mentioned compounds. This method comprises

a) reacting a compound having the formula II

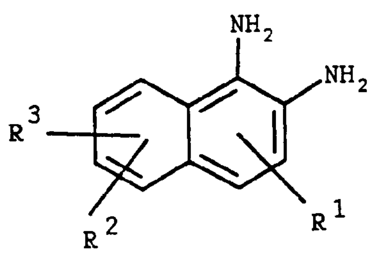

(II)

wherein $R^1$, $R^2$ and $R^3$ have the meanings defined above, with oxalate or a reactive derivative thereof to form a compound of formula I, or

b) refluxing a compound having the formula III

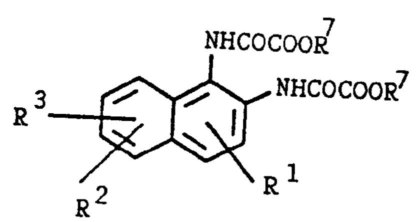

(III)

wherein $R^1$, $R^2$ and $R^3$ have the meanings defined above and $R^7$ is lower alkyl in a mineral acid, to form a compound of formula I, or

c) nitrating a compound having the formula IV

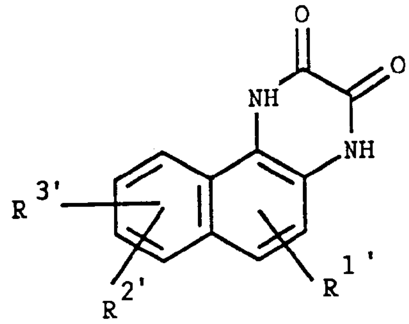

(IV)

wherein at least one of $R^{1'}$, $R^{2'}$ and $R^{3'}$ is hydrogen and the others are as defined for $R^1$, $R^2$ and $R^3$ respectively, to form a compound of formula I, or

d) reducing a compound having the formula V

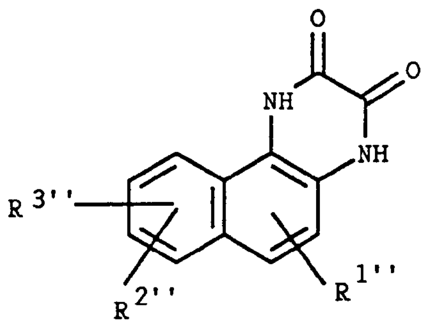

(V)

wherein at least one of $R^{1''}$, $R^{2''}$ and $R^{3''}$ is nitro and the others are as defined for $R^1$, $R^2$ and $R^3$ respectively, to form a compound of formula I, wherein at least one of $R^1$, $R^2$ and $R^3$ is amino, or

e) reacting a compound having the formula VI

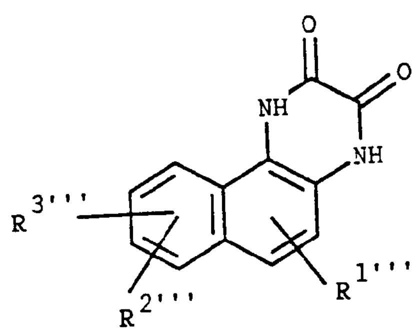

(VI)

wherein at least one of $R^{1'''}$, $R^{2'''}$, and $R^{3'''}$ is $N_2^+$ and the others are as defined for $R^1$, $R^2$ and $R^3$ respectively, with potassium tetracyanonickelate to form a compound of formula I, wherein at least one of $R^1$, $R^2$ and $R^3$ is CN.

The pharmacological properties of the compounds of the present invention can be illustrated by determining their capability for displacing radioactively labelled 2-amino3-hydroxy-5-methyl-4-isoxazolepropionic acid (AMPA) from the AMPA type receptors. The antagonistic properties of the compounds is demonstrated by their capability to antagonize quisqualic acid stimulated spreading depression in chicken retina.

The displacement activity of the compounds may be shown by determining the $IC_{50}$ value which represents the concentration (µg/ml) which causes a displacement of 50% of the specific binding of $^3$H-AMPA.

The antagonism is measured by determining the $IC_{50}$ value which represents the concentration which produces a 50% maximal inhibition of quisqualic acid stimulated spreading depression in chicken retina.

$^3$H-AMPA binding (Test 1)

500 µl of thawed rat cerebral cortical membrane homogenate in Tris-HCl (30 mM), $CaCl_2$ (2.5 mM) and KSCN (1OO mM) pH 7.1 were incubated at 0°C for 30 min. with 25 µl $^3$H-AMPA (5 nM final concentration) and the test compound and buffer. Nonspecific binding was determined by incubation with L-glutamic acid (600 µM final concentration). The binding reaction was terminated by adding 5 ml of ice-cold buffer followed by filtration through Whatman GF/C glass fibre filters and 2x5 ml wash with ice-cold buffer. Bound radioactivity was measured by scintillation counting. $IC_{50}$ was determined by Hill analysis of at least four concentrations of test compound.

Spreading depression (Test 2)

Chicks (3-10 days old) were decapitated, the eyes enucleated and sectioned along the equatorial plane. After removal of the anterior chamber and the vitreous body, the posterior chamber of each eye was placed in a small petri dish containing a physiological saline solution (P.S.S.) of the following composition (mM) NaCl (100), KCl (6.0), $CaCl_2$ (1.0), $MgS0_4$ (1.0), $NaHCO_3$ (30), $NaH_2PO_4$ (1.0), glucose (20).

The solution was saturated with 100% $O_2$ and maintained at 26°C.

The eyes are initially incubated in normal P.S.S. for 15-30 min. and then transferred to P.S.S. containing KCl (20 mM), NMDLA (30 µg/ml), quisqualate (1 µg/ml), kainate (1 µg/ml), or glycin (200 µg/ml). In these "stimulating solutions" S.D.s start spontaneously usually from the edge of the retina, and can be easily observed by eye. The time taken for an S.D. to start in each eye is measured.

After a further 15 minutes of incubation in normal P.S.S. the eyes are transferred to normal P.S.S. containing the test compound and incubated for 15 minutes. Thereafter the eyes are transferred to a "stimulating solution" containing the same concentration of the test compound. The time taken for an S.D. to start in each eye is again measured. The eyes are then placed back in normal P.S.S. and after 15 minutes the time taken for S.D. to start is again measured, in order to assess the degree of recovery from any drug effects.

An increase in the time taken for S.D. to start of 30 seconds more than the control time is considered 100% inhibition of S.D. The drug effects therefore are expressed as the percentage maximum response obtained for a given dose. The test value can be quoted therefore as the concentration (µg/ml) of test substance which produces a 50% maximal inhibition ($IC_{50}$).

Test results obtained by testing some compounds employed in the present invention will appear from the following table 1.

Table 1

| Compound of example | Test 1 $IC_{50}$ µg/ml | Test 2 $IC_{50}$ µg/ml |
|---|---|---|
| 8 | 0.55 | 1.3 |
| 11 | 0.17 | 0.54 |
| 14 | 0.07 | 0.41 |
| 21 | 0.16 | 1.0 |

The pharmaceutical preparations or compositions comprising the compounds of the invention may be administered to humans or animals by oral or parenteral route.

An effective amount of the active compound or a pharmaceutically acceptable salt thereof may be determined in accordance with the usual factors, such as the nature and severity of the condition and the weight of

the mammal requiring treatment.

Conventional excipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose, and polyvinylpyrrolidone.

The pharmaceutical preparations can be sterilized and mixed, if desired, with auxiliary agents, such as lubricants, preservatives, stabilizers, wetting agents, emulsifiers, salt for influencing osmotic pressure, buffers and/or colouring substances and the like, which do not deleteriously react with the active compounds.

Injectable solutions or suspensions, preferably aqueous solutions with the active compound dissolved in polyhydroxylated castor oil, are particularly suitable for parenteral administration.

Ampoules are convenient unit dosage forms.

Tablets, dragees, or capsules containing talc and/or a carrier or binder or the like are particularly suitable for oral administration. The carrier preferably is lactose and/or corn starch and/or potato starch.

A syrup, elixir, or the like can be used in the cases where a sweetened vehicle can be employed or is desired.

Generally, the compounds of this invention are dispensed in unit dosage form comprising 10-200 mg of active ingredient in or together with a pharmaceutically acceptable carrier per unit dosage.

The dosage of the compounds according to this invention is 1-500 mg/day, e.g. about 100 mg per dose, when administered to patients, e.g. humans, as a drug.

A typical tablet which may be prepared by conventional tabletting techniques contains:

<u>Core</u>:

| | |
|---|---|
| Active compound (as free compound or salt thereof) | 100 mg |
| Colloidal silicon dioxide (Aerosil®) | 1.5 mg |
| Cellulose, microcryst. (Avicel®) | 70 mg |
| Modified cellulose gum (Ac-Di-Sol®) | 7.5 mg |
| Magnesium stearate | 1 mg |

<u>Coating</u>:

| | | |
|---|---|---|
| HPMC | approx. | 9 mg |
| *Mywacett® 9-40 T | approx. | 0.9 mg |

* Acylated monoglyceride used as plasticizer for film-coating

The free quinoxaline compounds of the present invention which form alkali metal or alkaline earth metal salts may be employed in such salt form. Such alkali metal or earth alkali metal salts are ordinarily formed by reacting the quinoxaline compound with an equivalent amount or excess of the selected alkali metal or earth alkali metal as the hydroxide, frequently and suitably by admixture in the presence of a neutral solvent, from which the salt may be precipitated or recovered in other conventional manner, e.g. by evaporation. Administration of a compound of the invention is often preferably in the form of a pharmaceutically acceptable water-soluble alkali metal or earth alkali metal salt thereof, and orally, rectally, or parenterally in the form of a pharmaceutical composition wherein it is present together with a pharmaceutically acceptable liquid or solid carrier or diluent.

The compounds of the invention, together with a conventional adjuvant, carrier, or diluent, may be placed into the form of pharmaceutical compositions and unit dosages thereof, and in such form may be employed as

solids, such as tablets or filled capsules, or liquids, such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical composition and unit dosage forms thereof may comprise conventional ingredients in conventional proportions, with or without additional active compounds or principles, and such unit dosage forms may contain any suitable effective AMPA antagonistic amount of the active ingredient commensurate with the intended daily dosage range to be employed. Tablets containing 10 mg to 200 mg of active ingredient or, more specified 50 mg, per tablet, are accordingly suitable representative unit dosage forms.

Due to their high degree of AMPA antagonistic activity and their low toxicity, together presenting a most favourable therapeutic index, the compounds of the invention may be administered to a subject, e.g. a living animal body, in need of such treatment, elimination, alleviation, or amelioration of an indication which is sensitive to a change in the AMPA receptor condition, e.g. sclerosis, Parkinsonism, Alzheimer's disease, Huntington's disease, epilepsy, deficiences seen after ischemia, anoxia, hypoglycemia, head and spinal cord trauma, psychosis, muscle rigidity, emesis and analgesia, often preferably in the form of an alkali metal or earth alkali metal salt thereof, concurrently, simultaneously, or together with a pharmaceutically acceptable carrier or diluent, especially and preferably in the form of a pharmaceutical composition thereof, whether by oral, rectal, or parenteral (including subcutaneous) route, in an effective amount. Suitable dosage ranges are 10-200 milligrams daily, preferably 50-100 milligrams daily, and especially 70-100 milligrams daily, depending as usual upon the exact mode of administration, form in which administered, the indication toward which the administration is directed, the subject involved and the body weight of the subject involved, and the preference and experience of the physician or veterinarian in charge. Such method of treating may be described as the treatment of an indication caused by or related to hyperactivity of the excitatory neurotransmitters, and particularly the AMPA receptors, in a subject in need thereof, which comprises the step of administering to the said subject a neurologically effective amount of an AMPA antagonistic quinoxaline compound of the invention, or a pharmaceutically acceptable salt thereof.

The invention will now be described in further detail with reference to the following examples.

EXAMPLE 1

a. 1-Amino-4-bromo-5-nitronaphthalene

To an ice-cooled solution of 20.0 g (0.11 mol) 1-amino-5-nitronaphthalene in 500 ml dry dimethylformamide was added 19.0 g (0.11 mol) NBS. Stirring was continued at 25°C for 2 h. The reaction mixture was evaporated in vacuo, and the residue was stirred with water to give a crude product. Recrystallization (glacial acetic acid-water) gave 26.0 g (91%) of 1-amino-4-bromo-5-nitronaphthalene. M.p. 105°C.

b. 4-Bromo-5-nitro-1-sulfamoylnaphthalene

A solution of 15.0 (56.0 mmol) 1-amino-4-bromo-5-nitronaphthalene in 300 ml glacial acetic acid was added 30 ml concentrated hydrochloric acid, and then at 0°C di-azotized with a solution of 4.05 g (58.7 mmol) sodium nitrite in 30 ml water. Stirring was continued at 0°C for 1 h, and then the reaction mixture was added to a saturated solution of sulfur dioxide in 300 ml glacial acetic acid containing 1.0 g cupric chloride as a catalyst. Stirring was continued at 25°C for 1 h, and then the reaction mixture was poured into 1000 ml ice-water to give the sulfonyl chloride as a precipitate. Ammonia was bubbled through a solution of the crude product in 300 ml ethyl acetate. The reaction mixture was filtered and evaporated in vacuo. The residue was stirred with water to give 3.0 g (16%) of 4-bromo-5-nitro-1-sulfamoylnaphthalene.

c. 5-Amino-4-bromo-1-sulfamoylnaphthalene

A solution of 3.0 g (9.1 mmol) 4-bromo-5-nitro-1-sulfamoylnaphthalene in 200 ml ethyl acetate and 200 ml ethanol was hydrogenated at atm. pressure by using Ra-Ni (0.5 g) as a catalyst. The reaction mixture was filtered and evaporated in vacuo. The residue was stirred with water to give 2.5 g (91%) of 5-amino-4-bromo-1-sulfamoylnaphthalene.

d. 4-Bromo-5-(4-chlorophenylazo)-1-sulfamoylnaphthalene

A solution of 1.1 g (8.6 mmol) 4-chloroaniline in a mixture of 2 ml concentrated hydrochloric acid and 15 ml water was ice-cooled, and then diazotized with 0.63 g (8.9 mmol) sodium nitrite in 10 ml water. 2.5 g (8.2

mmol) 5-amino-4-bromo-1-sulfamoylnaphthalene was dissolved in 75 ml glacial acetic acid by heating, and then added 10 ml 2N sulfuric acid. The solution was cooled to ca. 50°C, and then the diazonium salt solution was added. Stirring was continued for a few minutes, and then a solution of 40 g sodium acetate in 100 ml water was added. The precipitated product was filtered off and washed with water. Recrystallization (ethanol-water) gave 2.06 g (58%) of 4-bromo-5-(4-chlorophenylazo)-1-sulfamoyl-naphthalene.

e. 10-Bromo-7-sulfamoylbenzo[f]quinoxaline-2,3(1H,4H)-dione

To a solution of 3.0 g (13.3 mmol) stannous chloride dihydrate in 25 ml concentrated hydrochloric acid was added 2.06 g (4.68 mmol) 4-bromo-5-(4-chlorophenylazo)-1-sulfamoylnaphthalene. The mixture was stirred at 70°C for 2 h. After cooling to 25°C, the mixture was poured into 25 ml ice-water, and the precipitate was filtered off. The crude 1,2-diamino derivative and 2.0 g oxalic acid dihydrate in 100 ml 4N hydrochloric acid was refluxed for 4 h. After cooling to 25°C, the precipitate was filtered off. Recrystallization (dimethylformamide-water) gave 0.75 g (45%) of 10-bromo-7-sulfamoylbenzo[f]quinoxaline-2,3(1H,4H)-dione, m.p. decomp. $^1$H-NMR (DMSO-$d_6$): δ 12.2 (2H,s), 8.8 (1H,d,-J=9.2 Hz), 8.75 (1H,d,J=9.2 Hz), 7.95 (2H,broad s), 7.7 (1H,d,J=9.2 Hz), 7.5 (1H,d,J=9.2 Hz).

f. 10-Bromo-6-nitro-7-sulfamoylbenzo[f]quinoxaline-2,3(1H,4H)-dione

To a solution of 0.5 g (1.34 mmol) 10-bromo-7-sulfamoylbenzo[f]quinoxaline-2,3(1H,4H)-dione in 25 ml concentrated sulfuric acid was added at 0°C 138 mg (1.35 mmol) potassium nitrate. Stirring was continued at 0°C for 30 min., and then the mixture was poured into 100 ml ice-water to give a precipitate. Recrystallization (dimethylformamide-water) gave 0.2 g (36%) of 10-bromo-6-nitro-7-sulfamoylbenzo[f]quinoxaline-2,3(1H,4H)-dione, m.p. 350°C decomp. $^1$H-NMR (DMSO-$d_6$): δ 12.5 (1H,s), 12.3 (1H,s), 8.8 (1H,d,J=9.5 Hz), 8.15 (1H,s), 7.95 (1H,s), 7.9 (1H,d,J=9.5 Hz), 7.6 (2H,broad s).

EXAMPLE 2

a. 2-Ethoxalylamino-6-(N-ethoxalyl)sulfamoylnaphthalene

To a solution of 7.7 g (34.7 mmol) 2-amino-6-sulfamoylnaphthalene in 250 ml dry tetrahydrofuran was added 21.0 ml (0.15 mol) dry triethylamine, and then dropwise 17.0 ml (0.15 mol) ethoxalylchloride. Stirring was continued at 25°C for 24 h. The reaction mixture was filtered and evaporated in vacuo. The residue was stirred with ethanol to give 13.1 g (90%) 2-ethoxalylamino-6-(N-ethoxalyl)sulfamoylnaphthalene, m.p. 191°C.

b. 2-Ethoxalylamino-6-(N-ethoxalyl)sulfamoyl-1-nitronaphthalene

To an ice-cooled mixture of 100 ml 100% nitric acid and 100 ml glacial acetic acid was added gradually 12.8 g (30.3 mmol) 2-ethoxalylamino-6-(N-ethoxalyl)sulfamoylnaphthalene. Stirring was continued at 0°C for 90 min. The reaction mixture was poured into 300 ml ice-water to give 13.1 g (90%) of 2-ethoxalylamino-6-(N-ethoxalyl)sulfamoyl-1-nitronaphthalene, m.p. 200°C decomp.

c. 2-Amino-1-nitro-6-sulfamoylnaphthalene

A solution of 5.0 g (10.7 mmol) 2-ethoxalylamino-6-(N-ethoxalyl)sulfamoyl-1-nitronaphthalene in a mixture of 75 ml concentrated hydrochloric acid, 75 ml water and 75 ml ethanol was refluxed for 2 h. The reaction mixture was cooled to 25°C to give 2.5 g (87%) of 2-amino-1-nitro-6-sulfamoylnaphthalene, m.p. 260°C decomp.

d. 8-Sulfamoylbenzo[f]quinoxaline-2,3(1H,4H)-dione

To a solution of 5.08 g (22.5 mmol) stannous chloride dihydrate in 50 ml concentrated hydrochloric acid was added 2.0 g (7.5 mmol) 2-amino-1-nitro-6-sulfamoylnaphthalene. The mixture was stirred at 70°C for 45 min. After cooling to 25°C, 100 ml ice-water was added to give a precipitate. The crude 1,2-diamino derivative was added to 2.5 g oxalic acid dihydrate in 50 ml 2N hydrochloric acid, and the mixture was refluxed for 3 h. After cooling to 25°C, the precipitate was filtered off. Recrystallization (dimethylformamide-water) gave 1.7 g (70%) of 8-sulfamoylbenzo[f]quinoxaline-2,3(1H,4H)-dione, m.p. >400°C.

e. 6-Nitro-8-sulfamoylbenzo[f]quinoxaline-2,3(1H,4H)-dione

To a solution of 0.5 g (1.7 mmol) 8-sulfamoylbenzo[f]-quinoxaline-2,3(1H,4H)-dione in 10 ml concentrated sulfuric acid was added at 0°C 0.075 ml (1.82 mmol) 100% nitric acid. Stirring was continued at 0°C for 1 h, and then the mixture was poured into 50 ml ice-water to give a precipitate. Recrystallization (dimethylformamide-methanol) gave 0.4 g (50%) 6-nitro-8-sulfamoylbenzo[f]quinoxaline-2,3(1H,4H)-dione, m.p. 380°C decomp. $^1$H- NMR (DMSO-$d_6$): δ 12.5 (2H, broad d), 9.0 (1H,s), 8.75 (1H,d), 8.25 (1H,s), 7.9 (1H,d), 7.5 (2H, broad s).

EXAMPLE 3

a. 2-Acetamido-6-(carbamoylmethyl)sulfonylnaphthalene

To a solution of 4.8 g (19.3 mmol) 6-acetamidonaphthalene-2-sulfinic acid in 50 ml dry dimethylformamide was added 0.72 g (19.5 mmol) 65% sodium hydride. After stirring at 25°C for 30 min., 3.54 g (19.5 mmol) iodoacetamide was added. Stirring was continued at 70°C for 30 min., and then the reaction mixture was evaporated in vacuo. Column chromatography (silica gel; eluent: ethyl acetate) gave 3.5 g (59%) of 2-acetamido-6-(carbamoylmethyl)sulfonylnaphthalene, m.p. 228°C decomp.

b. 2-Acetamido-6-(carbamoylmethyl)sulfonyl-1-nitronaphthalene

To an ice-cooled mixture of 20 ml glacial acetic acid and 20 ml 100% nitric acid was added 2.1 g (6.8 mmol) 2-acetamido-6-(carbamoylmethyl)sulfonylnaphthalene. After stirring at 0°C for 1 h, the reaction mixture was poured into 100 ml ice-water to give 2.1 g (84%) of 2-acetamido-6-(carbamoylmethyl)sulfonyl-1-nitronaphthalene, m.p. 245°C decomp.

c. 2-Amino-6-(carbamoylmethyl)sulfonyl-1-nitronaphthalene

A solution of 1.8 g (5.1 mmol) 2-acetamido-6-(carbamoylmethyl)sulfonyl-1-nitronaphthalene in 50 ml concentrated sulfuric acid was stirred at 25°C for 3 h. The reaction mixture was poured into 100 ml ice-water to give 1.47 g (93%) of 2-amino-6-(carbamoylmethyl)sulfonyl-1-nitronaphthalene, m.p. 254°C decomp.

d. 8-(Carbamoylmethyl)sulfonylbenzo[f]quinoxaline-2,3(1H,4H)-dione

A solution of 1.0 g (3.2 mmol) 2-amino-6-(carbamoylmethyl)sulfonyl-1-nitronaphthalene in a mixture of 200 ml ethyl acetate and 200 ml ethanol was hydrogenated at atm. pressure by using 0.5 Ra-Ni as a catalyst. The reaction mixture was filtered and evaporated in vacuo to give the 1,2-diamino derivative. The crude product and 1.5 g oxalic acid dihydrate in 50 ml 4N hydrochloric acid was refluxed for 2 h. After cooling to 25°C, the precipitate was filtered off and recrystallized (dimethylformamide-water) to give 0.53 g (50%) of 8-(carbamoylmethyl)sulfonylbenzo[f]quinoxaline-2,3(1H,4H)-dione, m.p. decomp.

e. 8-(Carbamoylmethyl)sulfonyl-6-nitro-benzo[f]quinoxaline-2,3(1H,4H)-dione

To an ice-cooled solution of 0.29 g (0.87 mmol) 8-(carbamoylmethyl)sulfonyl-benzo[f]quinoxaline-2,3(1H,4H)dione in 10 ml concentrated sulfuric acid was added 92 mg (0.91 mmol) potassium nitrate. After stirring at 0°C for 1 h, the reaction mixture was poured into 25 ml ice-water. The precipitate was filtered off and recrystallized (dimethylformamide-water) to give 0.17 g (52%) of 8-(carbamoylmethyl)sulfonyl-6-nitro-benzo[f]-quinoxaline-2,3(1H,4H)-dione, m.p. 370°C decomp. $^1$H-NMR (DMSO- $d_6$): δ 12.6 (2H,broad d), 9.2 (1H,s), 8.95 (1H,d,J=9.2 Hz), 8.4 (1H,s), 8.15 (1H,d,J=9.2 Hz), 4.7 (2H,s).

EXAMPLE 4

a. 6-Acetamidonaphthalene-1-sulfinic acid

To a solution of 4.4 g (35 mmol) sodium sulfite and 3.0 g (35 mmol) sodium hydrogencarbonate in 60 ml water was at 75°C added 5.0 g (17.6 mmol) 6-acetamidonaphthalene-1-sulfonyl chloride. Stirring was continued at 75°C for 1 h. After cooling to 25°C 4N hydrochloride acid was added to pH 1. The precipitate was filtered off to give 3.12 g (71%) of 6-acetamidonaphthalene-1-sulfinic acid.

b. 2-Acetamido-5-methylsulfonylnaphthalene

To a solution of 3.1 g (12.5 mmol) 6-acetamidonaphthalene-1-sulfinic acid in 60 ml dry dimethylformamide was added 0.46 g (12.5 mmol) 65% sodium hydride. After stirring at 25°C for 20 min., 1.0 ml (16.1 mmol) iodomethane was added. Stirring was continued at 70°C for 20 min., and then the reaction mixture was evaporated in vacuo. Column chromatography (silica gel; eluent: ethyl acetate) gave 2.5 g (76%) of 2-acetamido-5-methylsulfonylnaphthalene as an oil.

c. 2-Acetamido-1-nitro-5-methylsulfonylnaphthalene

To an ice-cooled mixture of 25 ml glacial acetic acid and 25 ml 100% nitric acid was added gradually 2.1 g (8.0 mmol) 2-acetamido-5-methylsulfonylnaphthalene. Stirring was continued at 0°C for 1 h, and then the reaction mixture was poured into 50 ml ice-water to give 1.79 g (83%) of 2-acetamido-1-nitro-5-methylsulfonylnaphthalene, m.p. 180°C decomp.

d. 2-Amino-1-nitro-5-methylsulfonylnaphthalene

A solution of 5.0 g (16.2 mmol) 2-acetamido-1-nitro-5-methylsulfonylnaphthalene in a mixture of 50 ml concentrated hydrochloric acid, 50 ml water and 50 ml ethanol was refluxed for 30 min. 100 ml water was added and the reaction mixture was cooled in an ice-bath to give 3.52 g (82%) of 2-amino-1-nitro-5-methylsulfonylnaphthalene, m.p. 210°C decomp.

e. 7-Methylsulfonylbenzo[f]quinoxaline-2,3(1H,4H)-dione

A solution of 2.5 g (9.4 mmol) 2-amino-1-nitro-5-methylsulfonylnaphthalene in a mixture of 180 ml tetrahydrofuran and 180 ml ethanol was hydrogenated at atm. pressure by using 1 g Ra-Ni as a catalyst. The reaction mixture was filtered and evaporated in vacuo to give the 1,2-diaminonaphthalene derivative. The crude product and 2.6 g oxalic acid dihydrate in 50 ml 4N hydrochloric acid was refluxed for 90 min. After cooling to 25°C the precipitate was filtered off and recrystallized (dimethylformamide-water) to give 2.1 g (80%) of 7-methylsulfonylbenzo[f]quinoxaline-2,3(1H,4H)-dione, m.p. 397°C. MS (m/e): 290 (80%, $M^+$).

f. 6-Nitro-7-methylsulfonylbenzo[f]quinoxaline-2,3-(1H,4H)-dione

To an ice-cooled solution of 0.5 g (1.72 mmol) 7-sulfamoylbenzo[f]quinoxaline-2,3(1H,4H)-dione in 15 ml concentrated sulfuric acid was added 182 mg (1.80 mmol) potassium nitrate. After stirring at 0°C for 30 min., the reaction mixture was poured into 25 ml ice-water to give a precipitate. Recrystallization (dimethylformamide-water)gave 0.5 g (86%) of 6-nitro-7-methylsulfonylbenzo[f]quinoxaline-2,3(1H,4H)-dione, m.p. 395°C. $^1$H-NMR (DMSO-$d_6$): δ 12.5 (2H,broad d), 8.9 (1H,d), 8.45 (1H,d), 8.1 (1H,s), 7.9 (1H,t), 3.45 (3H,s).

EXAMPLE 5

6-Nitro-9-sulfamoylbenzo[f]quinoxaline-2,3(1H,4H)-dione

The title compound was prepared from 2-amino-7-sulfamoylnaphthalene using the same procedures as described for 6-nitro-8-sulfamoylbenzo[f]quinoxaline-2,3(1H,4H)-dione. M.p. >400°C decomp. $^1$H-NMR (DMSO-$d_6$): δ 13.0 (1H,broad s), 12.5 (1H,broad s), 9.3 (1H,s), 8.75 (1H,d,J=10 Hz), 8.4 (1H,s), 8.1 (1H,d,J=10 Hz), 7.55 (2H,broad s).

EXAMPLE 6

7-Cyano-6-nitrobenzo[f]quinoxaline-2,3(1H,4H)-dione

To an ice-cooled solution of 0.4 g (1.69 mmol) 7-cyanobenzo[f]quinoxaline-2,3(1H,4H)-dione in 10 ml concentrated sulfuric acid was added 171 mg (1.69 mmol) potassium nitrate. After stirring at 0°C for 10 min., the reaction mixture was poured into 25 ml ice-water to give a precipitate. Recrystallization (dimethylformamide-water) gave 245 mg (52%) of 7-cyano-6-nitrobenzo[f]quinoxaline-2,3(1H,4H)-dione, m.p. 353°C decomp. $^1$H-NMR (DMSO- $d_6$): δ 12.7 (1H, broad s), 12.5 (1H,s), 9.05 (1H,d), 8.35 (1H,d), 8.1 (1H,s), 7.9 (1H,t).

EXAMPLE 7

6-Nitro-8-methylsulfonylbenzo[f]quinoxaline-2,3(1H,4H)-dione

The title compound was prepared from 6-acetamido-naphthalene-2-sulfonylchloride using the same procedures as described for 6-nitro-7-methylsulfonylbenzo[f]quinoxaline-2,3(1H,4H)-dione. M.p. 392°C decomp. $^1$H-NMR (DMSO-$d_6$): δ 12.5 (2H,broad d), 9.1 (1H,s), 8.85 (1H, d,J=9.6 Hz), 8.3 (1H,s), 8.1 (1H,d,J=9.6 Hz), 3.35 (3H,s).

EXAMPLE 8

a. 1-Nitro-5-trifluoromethylnaphthalene

A mixture of 10.0 g (39.7 mmol) 1-bromo-5-nitronapthalene, 20.0 g (147 mmol) trifluoromethylacetic acid sodium salt, 12.0 g (61.7 mmol) cuprous iodide and 200 ml 1-methyl-2-pyrrolidinone was stirred at 160°C for 20 h. After cooling to 25°C the mixture was poured into 1000 ml water and the precipitate was filtered off. The filter cake was stirred with 300 ml diethylether and then filtered. The filtrate was evaporated in vacuo and the residue was recrystallized (ethanol) to give 4.95 g 1-nitro-5-trifluoromethylnapthalene. (approx. 80 % pure).

b. 1-Amino-2-(4-chlorophenylazo)-5-trifluoromethylnapthalene

A solution of 6.00 g (80%) 1-nitro 5-trifluoromethylnapthalene in 200 ml ethanol was hydrogenated at atm. pressure by using Ra-Ni (2 g) as a catalyst. The reaction mixture was filtered and evaporated in vacuo. A solution of the residue in 80 ml water, 150 ml acetic acid and 80 ml concentrated sulfuric acid (95-97%) was stirred at 65°C. To the reaction mixture was added dropwise a diazonium salt solution prepared of 2.7 g (21.5 mmol) p-chloroaniline, 10 ml concentrated hydrochloric acid, 30 ml water (0°C) and 1.5 g (21.7 mmol) sodium nitrite. After 10 min. the mixture was cooled to 25°C and 100 g sodium acetate in 200 ml water was added. After stirring (1 h) the precipitate was filtered off, washed with water and recrystallized (ethanol) to give 2.3 g (26 %) 1-amino-2-(4-chlorophenylazo)-5-trifluoromethylnapthalene m.p.: 159.5°C (DSC)

c. 7-Trifluoromethylbenzo[f]quinoxaline-2,3(1H,4H)-dione

To a solution of 4.0 g (17.7 mmol) stannous chloride dihydrate in 50 ml concentrated hydrochloric acid was added 2.3 g (6.6 mmol) 1-amino-2-(4-chlorophenylazo)-5-trifluoromethylnapthalene. The mixture was stirred at 60°C for 30 min. After cooling to 25°C the pricipitate was filtered off. The precipitate, 4.0 g oxalic acid dihydrate and 80 ml 4N hydrochloric acid, was refluxed for 2 h. After cooling to 25°C the product was filtered off and washed with water, ethanol and diethylether to give 1.23 g (67 %) 7-trifluoromethylbenzo[f]-quinoxaline-2,3(1H,4H)-dione. M.p.: 401°C (DSC)

d. 6-Nitro-7-trifluoromethylbenzo[f]quinoxaline-2,3-(1H,4H)-dione

To an ice cooled solution of 0.75 g (2.7 mmol) of 7-trifluoromethylbenzo(f)quinoxaline-2,3(1H,4H)-dione in 25 ml concentrated sulfuric acid (95-97%) was added 0.29 g (2.9 mmol) potassium nitrate. Stirring was continued for 30 min. and then the mixture was poured into 200 ml ice-water. The pricipitate was filtered off and washed with water, ethanol and diethylether to give 0.70 g (80 %) 6-nitro-7-trifluoromethylbenzo[f]-quinoxaline-2,3(1H,4H)-dione. M.p.: decomp . MS m/e: 325 ($M^+$,100%) $^1$H-NMR (DMSO-$d_6$) :δ 12.60 (1H,broad s), 12.40 (1H,s), 9.00 (1H,d), 8.25 (1H,d), 8.15 (1H,s), 7.95 (1H,t)

EXAMPLE 9

a. 5-Acetyl-2-methoxy-1-nitrophthalene

An ice-cooled solution of 100% nitric acid (0.46 ml, 11 mmol) in 5 ml of acetic anhydride was added dropwise to a solution of 1-acetyl-6-methoxynaphthalene (2.2 g, 11 mmol) and 3 drops of conc. sulfuric acid in 75 ml of acetic anhydride while maintaining the temperature at -10°C. The mixture was stirred for 30 min. and poured into 300 ml of ice/water. The separated solid was filtered and washed with water and cold ethanol to give 2.0 g (74%) of the title compound, m.p. 135-138°C. $^1$H-NMR ($CDCl_3$): δ 2.66 (s,3H,$CH_3$), 3.93 (s,3H,$OCH_3$), 7.1-7.9 (m,4H,ArH), 8.77 (d,J=9 Hz, 1H, ArH).

b. 5-Acetyl-2-amino-1-nitronaphthalene

A solution of 5-acetyl-2-methoxy-1-nitronaphthalene (2.0 g, 8.1 mmol) in approximately 50 ml of liquid ammonia was heated at 100°C for 16 h in a teflon-lined steel bomb. The bomb was cooled, and excess ammonia was allowed to evaporate. The residue was taken up in 100 ml of ethyl acetate and washed with 100 ml of 1 N hydrochloric acid. The aqueous phase was extracted with ether (3 x 75 ml). The combined organic phases were washed with water (2 x 75 ml), dried and evaporated in vacuo to yield 1.5 g of crude product. Chromatography on silica (1.5% methanol in dichloromethane) afforded 0.76 g (40%) of the pure title compound, m.p. 170-172°C. $^1$H-NMR (DMSO-$d_6$): 2.67 (s,3H,$CH_3$), 7.15 (d,J=9 Hz,1H,ArH), 7.33-8.47 (m,6H,ArH+$NH_2$).

c. 7-Acetylbenzo[f]quinoxaline-2,3(1H,4H)-dione

A solution of stannous chloride dihydrate (2.25 g, 10 mmol) in 5 ml of conc. hydrochloric acid was added to a stirred suspension of 5-acetyl-2-amino-1-nitronaphthalene (0.58 g, 2.5 mmol) in 10 ml of methanol, and the mixture was heated at reflux for 1.5 h. To the hot mixture was added 5 ml of water and oxalic acid dihydrate (0.5 g, 4 mmol), and heating at reflux was continued for 2 h. The mixture was cooled and the precipitated solid was isolated by filtration, washed with water, ethanol and ether to give 0.50 g (78%) of the title compound, m.p. >350°C (DSC). 1H-NMR (DMSO-$d_6$):δ 2.70 (s,3H, $CH_3$), 7.22-8.76 (m,5H,ArH), 12.2 (broad s,2H, 2NH); MS m/e: 254 ($M^+$, 100%).

d. 7-Acetyl-6-nitrobenzo[f]quinoxaline-2,3(1H,4H)-dione

Finely powdered potassium nitrate (51 mg, 0.5 mmol) was added to a stirred solution of 7-acetylbenzo[f]quinoxaline-2,3(1H,4H)-dione (127 mg, 0.5 mmol) in 2 ml of conc. sulfuric acid. The mixture was stirred at room temperature for 30 min. and then poured into 50 ml of ice/water. The precipitated product was collected by filtration, washed with water and a small amount of ethanol and ether to give 82 mg (55%) of the title compound, m.p. 359°C decomp. (DSC). $^1$H-NMR (DMSO-$d_6$): δ 2.77 (s,3H,$CH_3$), 7.53-8.80 (m,4H,ArH), 12.2 (broad s, 2H,2NH).

EXAMPLE 10

a. 6-Acetyl-2-methoxy-1-nitronaphthalene

This compound was obtained from 2-acetyl-6-methoxynaphthalene (2.0 g, 10 mmol) as described for 5-acetyl-2-methoxy-1-nitronaphthalene affording 2.1 g (86%) pure product, m.p. 140-145°C. $^1$H-NMR ($CDCl_3$): δ 2.70 (s,3H, $CH_3$), 4.06 (s,3H,$OCH_3$), 7.41 (d,J=9 Hz,1H,ArH), 7.71 (d,J=9 Hz,1H,ArH), 8.09 (d,J=9 Hz,1H,ArH), 8.13 (dd, J=9,ca.1Hz,1H,ArH), 8.44 (d,J=ca.1Hz,1H,ArH).

b. 6-Acetyl-2-amino-1-nitronaphthalene

A solution of 6-acetyl-2-methoxy-1-nitronaphthalene (2.0 g, 8.1 mmol) in 25 ml of dry DMSO saturated with ammonia was heated with stirring at 100°C for 17 h in a teflon-lined steel bomb. The bomb was allowed to cool, and the mixture was poured into 200 ml of water. The precipitated product was collected by filtration and washed with water and cold methanol affording 1.75 g of crude title compound (containing ca. 35% of starting material), which was used without further purification in the next step.

c. 8-Acetylbenzo[f]quinoxaline-2,3(1H,4H)-dione

This compound was obtained from crude 6-acetyl-2-amino-12-nitronaphthalene (1.61 g) as described for 7-acetylbenzo[f]quinoxaline-2,3(1H,4H)-dione. The crude product was dissolved in 20 ml of hot DMF, treated with decolourizing charcoal and filtered. Then 25 ml of methanol was added dropwise with stirring on an ice bath, and the resulting precipitate was collected. The product was boiled in 10 ml of methanol for 5 min, isolated by filtration and dried in vacuo for 6 h to give 0.73 g (41%) of the title compound, m.p. 371°C (DSC). $^1$H-NMR (DMSO): δ 2.69 (s,3H,$CH_3$), 7.42 (d,J=9 Hz,1H,ArH), 7.87 (d,J=9 Hz,1H,ArH), 7.96-8.65 (m,3H,ArH), 12.22 (s,1H,NH), 12.28 (s,1H,NH).

d. 8-Acetyl-6-nitrobenzo[f]quinoxaline-2,3(1H,4H)-dione

Finely powdered potassium nitrate (210 mg, 2.1 mmol) was added to a solution of 8-acetylbenzo[f]quinoxaline-2,3(1H,4H)-dione (0.51 g, 2 mmol) in 5 ml of conc. sulfuric acid with stirring at 0°C. The mixture was stirred at 0°C for 70 min. and poured into 75 ml of water. The precipitated product was isolated by filtration, washed with water and methanol and recrystallized from DMF/methanol. The product was boiled in 30 ml of water for 10 min., cooled, filtered and dried in vacuo at 100°C to give 300 mg (50%) of the pure title compound, m.p. 366°C decomp. (DSC). $^1$H-NMR (DMSO-$d_6$): δ 2.67 (s,3H,$CH_3$), 8.05 (d,J=9 Hz,1H,ArH), 8.17 (s,1H,ArH), 8.65 (d,J=9 Hz),1H,ArH), 9.02 (s,1H,ArH), 12.35 (s,1H, NH), 12.50 (s,1H,NH), MS m/s: 299 ($M^+$, 65%).

EXAMPLE 11

a. 6-Amino-8-sulfamoylbenzo[f]quinoxaline-2,3(1H,4H)-dione hydrochloride

A solution of 6-nitro-8-sulfamoylbenzo[f]quinoxaline-2,3(1H,4H)-dione (2.0 g, 5.9 mmol) in 100 ml of DMF was hydrogenated at room temperature and atmospheric pressure in the presence of 5% palladium on carbon (500 mg) for 2 h. The catalyst was filtered off and washed with DMF. The filtrate, to which conc. hydrochloric acid (5 ml) had been added, was evaporated to dryness in vacuo. The residue was triturated with a mixture of conc. hydrochloric acid (5 ml) in ethanol (50 ml), filtered and dried to give 2.0 g (98%) of the title compound as a hydrate. $^1$H-NMR (DMSO-$d_6$): δ 5.2 (very broad s, $NH_2$.HCl + $XH_2O$), 7.23 (s,1H,ArH), 7.46 (broad s,2H, $SO_2NH_2$), 7.98 (d,J=9 Hz,1H,ArH), 8.56 (s,1H,ArH), 8.69 (d,J=9 Hz,1H,ArH), 12.1 (broad s,1H,NH), 12.3 (s,1H,NH).

b. 6-Cyano-8-sulfamoylbenzo[f]quinoxaline-2,3(1H,4H)-dione

A solution of 6-amino-8-sulfamoylbenzo[f]quinoxaline-2,3(1H,4H)-dione hydrochloride (3.43 g, 10 mmol) in 10 ml of conc. sulfuric acid was poured into 100 ml of ice/water. The resulting suspension was diazotised with sodium nitrite (0.76 g, 11 mmol) in 10 ml of water with stirring at 0°C. After stirring at 0°C for 1 h a solution of potassium tetracyanonickelate (6.6 g) and sodium hydrogen carbonate (30.3 g) in 250 ml of water was added dropwise during 25 min. Then the mixture was stirred at 30-40°C for 2 h and finally heated at 90°C for ca. 10 min. Conc. hydrochloric acid (25 ml) was added to the cooled mixture and the separated solid was isolated by filtration and washed with water. The crude product was chromatographed on silica gel with 10% acetic acid in ethyl acetate, and recrystallized from DMF/methanol. The product was boiled for 30 min. in 50 ml of water, isolated by filtration, and dried in vacuo at 150°C to give 0.53 g (17%) of the pure title compound, m.p. 397°C (DSC); IR (KBr): 2236 (CN), 1702 (C=O) $cm^{-}$; $^1$H-NMR (DMSO-$d_6$): δ 7.59 (s,2H,$SO_2NH_2$), 7.92 (s,1H,ArH), 8.00 (d,J=9 Hz,1H,ArH), 8,46 (s,1H, ArH), 8.90 (d,J=9 Hz,1H,ArH), 12.5 (broad s,1H,NH, only one exchangeable proton could be seen); MS m/e: 316 ($M^+$, 78%).

EXAMPLE 12

a. 7,9-Disulfamoylbenzo[f]quinoxaline-2,3(1H,4H)-dione

A solution of 7-sulfamoylbenzo[f]quinoxaline-2,3(1H,4H)-dione (3.0 g, 10.3 mmol) in chlorosulfonic acid (10 ml) was stirred at room temperature for 2 h, and then at 120°C for 3 h. The cooled reaction mixture was cautiously added dropwise to 100 g of ice. The precipitated solid was collected by filtration, dissolved in ethyl acetate (250 ml), washed with water (3 x 100 ml), dried, and evaporated to dryness. The crude disulfochloride was dissolved in dry tetrahydrofuran (50 ml) and treated with ammonia. After 2 h at room temperature, the mixture was evaporated to dryness, and the residue was dissolved in 1N sodium hydroxide (50 ml), treated with charcoal, filtered, and reprecipitated with 4M hydrochloric acid to give 0.78 g (20%) of the title compound, m.p. >350°C (DSC). $^1$H-NMR (DMSO-$d_6$): δ 7.62 (d,J=9 Hz,1H,ArH), 7.65 (s,2H, $SO_2NH_2$), 7.88 (s,2H,$SO_2NH_2$), 8.42 (d,J=9 Hz,1H,ArH), 8.49 (s,1H,ArH), 9.42 (s,1H,ArH), 12.32 (s,1H,NH), 12.72 (s,1H,NH); MS m/e: 370 ($M^+$, 1%).

b. 6-Nitro-7,9-disulfamoylbenzo[f]quinoxaline-2,3-(1H,4H)-dione

A solution of 100% nitric acid (50 μl, 1.2 mmol) in conc. sulfuric acid (0.5 ml) was added dropwise to a solution of 7,9-disulfamoylbenzo[f]quinoxaline-2,3-(1H,4H)-dione (370 mg, 1 mmol) in conc. sulfuric acid (5 ml) with stirring at 0°C. After 3 h at 0°C the mixture was poured onto 25 g of ice, and the precipitated solid was collected by filtration and washed with water. Recrystallization from DMF/methanol, followed by trituration with

boiling water and drying in vacuo, afforded 66 mg (16%) of the title compound, m.p. 383°C (decomp.) (DSC), IR (KBr): 1721, 1650 $cm^{-1}$. $^1$H-NMR (DMSO-$d_6$): δ 7.53 (broad s,4H,2 $SO_2NH_2$), 8.15 (s,1H,ArH), 8.69 (s,1H,ArH), 9.33 (s,1H,ArH), 12.4 (broad s,1H,NH), ca. 13 (very broad s,1H,NH).

## EXAMPLE 13

### a. 6-Cyano-2-methoxy-1-nitronaphthalene

Cuprous cyanide (11.8 g, 131.6 mmol) was added to a solution of 6-bromo-2-methoxy-1-nitronaphthalene (18.4 g, 65.8 mmol) in 70 ml of dry DMF, and the mixture was stirred at 150°C for 9 h. A solution of ferric chloride hexahydrate (35 g of $FeCl_3 \cdot 6H_2O$ and conc. hydrochloric acid (8.5 ml)) in water (52 ml) was added to the cooled mixture, and heating was continued at 60°C for 1 h. The cooled mixture was poured into water (900 ml) and the precipitated solid was filtered, and washed with water. Recrystallization from ethanol gave 10.5 g (70%) of the title compound, m.p. 209-211°C. $^1$H-NMR (DMSO-$d_6$): δ 4.08 (s,3H,$CH_3$), 7.5-8.7 (m,5H,ArH); IR (KBr): 2226 $cm^{-1}$ (CN).

### b. 2-Amino-6-cyano-1-nitronaphthalene

A solution of 6-cyano-2-methoxy-1-nitronaphthalene (7.5 g, 33 mmol) in dry DMSO was saturated with dry ammonia in a pressure vessel, and heated at 100°C for 3 h. The mixture was poured into 400 ml of water and the yellow precipitate was isolated by filtration, dried, and chromatographed on silica gel with dichloromethane/methanol (9:1) to yield 5.9 g (84%) of sufficiently pure product, m.p. 263-266°C; IR (KBr): 2228 (CN), 1644 $cm^{-1}$. $^1$H-NMR (DMSO-$d_6$): δ 7.27 (d,J=9 Hz,1H,ArH), 7.85 (d,J=9 Hz,1H,ArH), 7.94 (d,J=9 Hz,1H,ArH), 8.21 (broad s,2H,$NH_2$), 8.36 (s,1H,ArH), 8.53 (d,J=9 Hz,1H, ArH).

### c. 1,2-Diamino-6-cyanonaphthalene

A suspension of 2-amino-6-cyano-1-nitronaphthalene (0.75 g, 3.54 mmol) in abs. ethanol (150 ml) was hydrogenated at atmospheric pressure and room temperature in the presence of 5% palladium on carbon (150 mg) until the theoretical amount of hydrogen was taken up. The catalyst was removed by filtration, and the filtrate was evaporated to dryness to give 0.60 g (93%) of the diamine; IR (KBr): 2221 (CN), 1626 $cm^{-1}$. $^1$H-NMR (DMSO-$d_6$): δ 4.99 (broad s,2H,$NH_2$), 5.26 (broad s,2H,-$NH_2$), 7.06 (d,J=9 Hz,1H,ArH), 7.18 (d,J=9 Hz,1H,ArH), 7.41 (dd,J=9,ca.2 Hz,1H,H-7), 7.98 (d,J=9 Hz,1H,H-8), 8.13 (d,J=ca.2 Hz,1H,H-5).

### d. 8-Cyanobenzo[f]quinoxaline-2,3(1H,4H)-dione

A solution of ethyl oxalylchloride (7.9 ml, 70.8 mmol) in dry tetrahydrofuran (20 ml) was added dropwise to a solution of crude 1,2-diamino-6-cyanonaphthalene (4.3 g, 23.6 mmol) and dry triethylamine (9.8 ml, 70.8 mmol) in dry tetrahydrofuran (100 ml). The mixture was stirred at room temperature over night, filtered, and the filtrate evaporated to dryness. The oily residue (containing the 6-cyano-1,2-diethoxalylaminonaphthalene) was refluxed with stirring for 2 h in 4M hydrochloric acid (100 ml). The hot mixture was filtered, and the precipitate was washed with water and ethanol to give 3.1 g (55%) of the title compound, m.p. >375°C (DSC); IR (KBr): 2230 (CN), 1686 $cm^{-1}$. $^1$H-NMR (DMSO-$d_6$): δ 7.45 (d,J=9 Hz,1H,ArH), 7.77 (d,J=9 Hz,1H,ArH), 7.82 (d,J=9 Hz,1H,ArH), 8.51 (s,1H,ArH), 8.66 (d,J=9 Hz,1H, ArH), 12.25 (s,1H,NH), 12.28 (s,1H,NH).

### e. 8-Cyano-6-nitrobenzo[f]quinoxaline-2,3(1H,4H)-dione

8-Cyanobenzo[f]quinoxaline-2,3(1H,4H)-dione (3.0 g, 12.6 mmol) was dissolved in conc. sulfuric acid (300 ml) with stirring at 0°C. Finely powdered potassium nitrate (1.41 g, 14 mmol) was added to the solution, and stirring was continued for 15 min. at 0°C. The mixture was poured into water (2.5 l), and the precipitated product was collected by filtration, washed with water and ethanol to give the title compound in a yield of 1.56 g (44%), m.p. 396°C decomp. (DSC); IR (KBr): 2233 (CN), 1694 $cm^{-1}$. $^1$H-NMR (DMSO-$d_6$): δ 7.89 (d,J=9 Hz,1H,ArH), 8.23 (s,1H,ArH), 8.67 (d,J=9 Hz,1H,ArH), 8.85 (s,1H,ArH), 12.4 (s,1H,NH), 12.55 (broad s,1H,NH).

EXAMPLE 14

a. 8-Carbamoyl-2-methoxy-1-nitronaphthalene

To a stirred solution of 6-cyano-2-methoxy-1-nitronaphthalene (500 mg, 2.2 mmol) in DMSO (8 ml) was added potassium carbonate (50 mg) and dropwise 30% hydrogen peroxide (0.3 ml). After stirring at room temperature for 20 min., water (20 ml) was added, and the solid was isolated by filtration. Purification by silica gel chromatography with ethyl acetate afforded 290 mg (54%) of the title compound, m.p. 245-250°C. $^1$H-NMR (DMSO-$d_6$): δ 4.07 (s,3H,$CH_3$), 7.53 (broad s,1H,NH), 7.62 (d,J=9 Hz,1H,ArH), 7.76 (d,J=9 Hz,1H,ArH), 8.11 (d,J=9 Hz, 1H, ArH), 8.17 (broad s,1H,NH), 8.31 (d,J=9 Hz,1H, ArH), 8.59 (s,1H,ArH).

b. 8-Carbamoyl-6-nitrobenzo[f]quinoxaline-2,3(1H,4H)-dione

This compound was obtained from 8-carbamoyl-2-methoxy-1-nitronaphthalene according to the procedure described for 8-cyano-6-nitrobenzo[f]quinoxaline-2,3(1H,4H)-dione, m.p. >400°C decomp. (DSC); IR (KBr): 3372, 1706, 1659 $cm^{-1}$. $^1$H-NMR (DMSO-$d_6$): δ 7.66 (broad s,1H,NH), 8.14 (d,J=9 Hz,1H,ArH), 8.26 (broad s,1H,NH), 8.28 (s,1H,ArH), 8.78 (d,J=9 Hz,1H,ArH), 9.08 (s,1H,ArH), 12.40 (broad s,1H,NH), 12.58 (broad s,1H,NH).

EXAMPLE 15

6-Chloro-8-sulfamoylbenzo[f]quinoxaline-2,3(1H,4H)-dione

A suspension of 6-amino-8-sulfamoylbenzo[f]quinoxaline-2,3(1H,4H)-dione hydrochloride (0.69 g, 2 mmol) in 37%hydrochloric acid (10 ml) was diazotised with sodium nitrite (0.15 g, 2.17 mmol) in water (2 ml) with stirring at 0°C. After 1 h the mixture was poured into a freshly prepared solution of cuprous chloride (2.6 mmol) in 37% hydrochloric acid (10 ml) with stirring at 0°C. The mixture was then stirred at room temperature for 1 h and at 100°C for 30 min. After cooling the solid was isolated by filtration, washed with water and recrystallized from DMF/methanol with decolourising charcoal to give 131 mg (20%) of the title compound, m.p. 370°C decomp. (DSC). $^1$H-NMR (DMSO-$d_6$): δ 7.54 (broad s,2H,$SO_2NH_2$), 7.59 (s,1H,H-5), 7.96 (dd,J=9,ca. 2Hz, 1H,H-9), 8.56 (d,J=ca.2Hz,1H,H-7), 8.77 (d,J=9 Hz,1H,- H-10), 12.30 (s,1H,NH), 12.35 (s,1H,NH).

EXAMPLE 16

9-Methylsulfonyl-6-nitrobenzo[f]quinoxaline-2,3(1H,4H) dione

This compound was prepared from 7-acetylamino-2-naphthalenesulfonyl chloride as described for 7-methylsulfonyl-6-nitrobenzo[f]quinoxaline-2,3(1H,4H)-dione, m.p. 366°C decomp. (DSC). $^1$H-NMR (DMSO-$d_6$): δ 3.38 (s,3H,- $CH_3$), 8.11 (d,J=9 Hz,1H,ArH), 8.37 (s,1H,ArH), 8.74 (d,J=9 Hz,1H,ArH), 9.39 (s,1H,ArH), 12.45 (s,1H,NH), 12.9 (broad s,1H,NH).

EXAMPLE 17

a. 8-(3-(2-Oxo-1-pyrrolidinyl)propylsulfamoyl)benzo[f]quinoxaline-2,3(1H,4H)-dione

8-Sulfamoylbenzo[f]quinoxaline-2,3(1H,4H)-dione (1.46 g, 5 mmol) was added portion-wise to chlorosulfonic acid (15 ml) with stirring at room temperature. Then a catalytic amount of dry DMF was added, and the mixture was stirred over night at room temperature. The cooled solution was added dropwise to ice (150 g), and the precipitated solid was isolated by filtration, washed with water, and dried. To a filtered solution of the crude 8-chlorosulfonylbenzo[f]quinoxaline-2,3(1H,4H)-dione in tetrahydrofuran (150 ml) was added 1-(3-amino-propyl)-2-pyrrolidinone (4 ml, 28.5 mmol), and the mixture was stirred over night at room temperature. The mixture was evaporated to dryness in vacuo and the residue was tritutated with 1N hydrochloric acid. The product was isolated by filtration, washed with water and recrystallized from DMF/methanol afferding 1.1 g (53%) of the title compound, m.p. 277°C (DSC). $^1$H-NMR (DMSO-$d_6$): δ 1.54 (m,2H,$CH_2$), 1.82 (m,2H,$CH_2$), 2.13 (t,J=8 Hz,2H,$CH_2$), 2.71 (distorted q, 2H,$CH_2$), 3.11 (t,J=8 Hz,2H,$CH_2$), 3.20 (t,J=8 Hz,2H,$CH_2$), 7.48 (d,J=9 Hz,1H,ArH), 7.64 (t,J=6 Hz,1H,$SO_2NH$), 7.82 (d,J=9 Hz, 1H,ArH), 7.91 (d,J=9 Hz, 1H,ArH), 8.38 (s,1H,ArH), 8.74 (d,J=9 Hz,1H,ArH), 12.3 (s,2H,2NH).

b. 6-Nitro-8-(3-(2-oxo-1-pyrrolidinyl)propylsulfamoyl)benzo[f]quinoxaline-2,3(1H,4H)-dione

A solution of 8-(3-(2-Oxo-1-pyrrolidinyl)propylsulfamoyl)benzo[f]quinoxaline-2,3(1H,4H)-dione (0.15 g, 0.36 mmol) in conc. sulfuric acid (7 ml) was nitrated with powdered potassium nitrate (40 mg, 0.4 mmol) with stirring at 0°C for 90 min. Usual workup afforded 128 mg (77%) of the title compound, m.p. >275°C decomp. (DSC). $^1$H-NMR (DMSO-$d_6$): δ 1.56 (m,2H,$CH_2$), 1.84 (m, 2H,$CH_2$), 2.14 (t,J=8 Hz,2H,$CH_2$), 2.76 (distorted q,2H, $CH_2$), 3.12 (t,J=8 Hz, 2H,$CH_2$), 3.22 (t,J=8 Hz,2H,$CH_2$), 7.87 (distorted t,J=ca.6 Hz,1H,$SO_2$NH), 7.98 (d,J=9 Hz,1H, ArH), 8.39 (s,1H,ArH), 8.92 (d,J=9 Hz,1H,ArH), 9.05 (s,1H,ArH), 12.45 (s,1H,NH), 12.64 (s,1H,NH).

EXAMPLE 18

8-Methylsulfamoyl-6-nitrobenzo[f]quinoxaline-2,3(1H,4H)-dione

This compound was obtained as described above, by reaction of 8-chlorosulfonylbenzo[f]quinoxaline-2,3(1H,4H)-dione with methylamine followed by nitration with potassium nitrate in conc. sulfuric acid, m.p. 358°C decomp. (DSC). $^1$H-NMR (DMSO): δ 2.47 (d,J=5 Hz,3H,$CH_3$), 7.70 (q,J=5 Hz,1H,$SO_2$NH), 7.94 (dd,J=9, 1.8 Hz,1H,H-9), 8.39 (s,1H,H-5), 8.92 (d,J=9 Hz,1H,H-10), 9.04 (d,J=1.6 Hz,1H,H-7), 12.45 (broad s,1H,NH), 12.64 (broad s,1H,NH).

EXAMPLE 19

8-Ethylsulfamoyl-6-nitrobenzo[f]quinoxaline-2,3(1H,4H)-dione

This compound was obtained as described above, by reaction of 8-chlorosulfonylbenzo[f]quinoxaline-2,3(1H,4H)-dione with ethylamine followed by nitration with one equivalent of 100% nitric acid in conc. sulfuric acid, m.p. 357°C decomp (DSC). $^1$H-NMR (DMSO-$d_6$): δ 0.98 (t,J=7 Hz,3H,$CH_3$), 2.86 (q,J=7 Hz,2H,$CH_2$), 7.80 (t,J=5 Hz,1H,$SO_2$NH), 8.01 (d,J=9 Hz,1H,H-9), 8.39 (s,1H,H-5), 8.92 (d,J=9 Hz,1H,H-10), 9.05 (s,1H,H-7), 12.50 (broad s,1H,NH), 12.65 (broad s,1H,NH).

EXAMPLE 20

8-Dimethylsulfamoyl-6-nitrobenzo[f]quinoxaline-2,3-(1H,4H)-dione

This compound was prepared as described above by reaction of 8-chlorosulfonylbenzo[f]quinoxaline-2,3(1H,4H)-dione with dimethylamine followed by nitration with potassium nitrate in conc. sulfuric acid, m.p. 357°C decomp. (DSC). $^1$H-NMR (DMSO-$d_6$): δ 2.69 (s,6H,2$CH_3$), 7.95 (distorted d,J=9 Hz,1H,H-9), 8.40 (s,1H,H-5), 8.95 (d,J=9 Hz,1H,H-10), 9.01 (s,1H,H-7), 12.50 (broad s, 1H,NH), 12.75 (broad s,1H,NH).

EXAMPLE 21

8-Carbamoylmethylsulfamoyl-6-nitrobenzo[f]quinoxaline-2,3(1H,4H)-dione

This compound was prepared as described above, except that the solution of 8-chlorosulfonylbenzo[f]quinoxaline-2,3(1H,4H)-dione in tetrahydrofuran was added an aqueous solution of glycinamide hydrochloride followed by the addition of 0.5 mol eqv. of sodium carbonate. After stirring at room temperature for 2 h, the product was worked up and nitrated in the usual way, m.p. >260°C decomp. (DSC). $^1$H-NMR (DMSO-$d_6$): δ 3.46 (d,J= ca. 5Hz,2H,$CH_2$), 7.05 and 7.30 (broad singlets,2H, $CONH_2$), 8.02 (d,J=9 Hz,1H,H-9), 8.17 (t,J=ca.5Hz,1H, $SO_2$NH), 8.38 (s,1H,H-5), 8.90 (d,J=9 Hz,1H,H-10), 9.07 (s,1H,H-7), 12.47 (broad s,1H,NH), 12.65 (broad s,1H, NH).

EXAMPLE 22

9-Methylsulfamoyl-6-nitrobenzo[f]quinoxaline-2,3(1H,4H)-dione

This compound was obtained as described for 8-(3-(2-oxo-1-pyrrolidinyl)propylsulfamoyl)benzo[f]quinoxaline-2,3(1H,4H)-dione, except that 9-sulfamoylbenzo[f]quinoxaline-2,3(1H,4H)-dione was reacted with chlorosulfonic acid. The crude sulfonyl chloride was then reacted with methylamine, and finally nitrated in the usual

way to give the title compound, m.p. 373°C decomp. (DSC). $^1$H-NMR (DMSO-$d_6$): δ 2.51 (d,J=5 Hz,3H,$CH_3$), 7.60 (q,J=5 Hz,1H,$SO_2NH$), 7.98 (dd,J=9,ca.2 Hz,1H,H-8), 8.36 (s,1H,H-5), 8.73 (d,J=9 Hz,1H,H-7), 9.25 (d,J=ca. 2Hz,1H,H-10), 12.42 (s,1H,NH), 13.05 (s,1H,NH).

## Claims

1. Quinoxaline compounds having the formula I

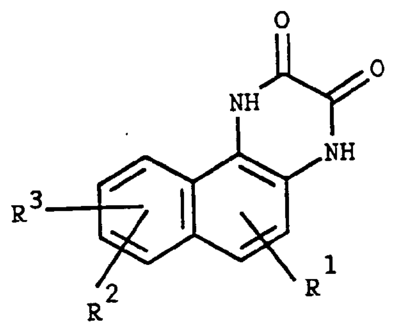

(I)

wherein
$R^1$ is hydrogen, $NO_2$, CN, $CF_3$ or halogen, $R^2$ and $R^3$ independently are hydrogen, CN, $CF_3$, halogen, $C(NOH)C_{1-6}$-alkyl, $COR^4$ or $SO_2R^4$, wherein $R^4$ is $C_{1-6}$-alkyl, optionally substituted with $CONH_2$, or $NR^5R^6$, wherein $R^5$, $R^6$ independently are hydrogen, $C_{3-6}$-cycloalkyl, $C_{1-6}$-alkyl optionally substituted with $CONH_2$ or 2-oxo-pyrrolidinyl, or pharmaceutically acceptable salts thereof.

2. A compound according to claim 1 which is 6-nitro-8-sulfamoylbenzo-[f]quinoxaline-2,3(1H,4H)-dione.

3. A compound according to claim 1 which is 8-(carbamoyl-methyl)sulfonyl-6-nitrobenzo[f]quinoxaline-2,3(1H,4H)-dione.

4. Compounds according to claim 1 which is 6-cyano-8-sulfamoylbenzo[f]quinoxaline-2,3(1H,4H)-dione, 8-carbamoyl-6-nitrobenzo[f]quinoxaline-2,3(1H,4H)-dione.

5. A pharmaceutical composition comprising as active component a compound according to claim 1 or a pharmaceutically acceptable salt thereof and a pharmaceutically acceptable carrier or diluent.

6. A pharmaceutical composition according to claim 5 in the form of a dosage unit containing about 10-200 mg of the active compound.

7. A pharmaceutical composition suitable for use in the treatment of an indication related to hyperactivity of the excitatory neurotransmitters, which comprises as active component a compound according to claim 1 or a pharmaceutically acceptable salt thereof and a pharmaceutically acceptable carrier or diluent.

8. A method of treating an indication related to hyperactivity of the excitatory neurotransmitters in a subject in need thereof, which comprises the step of administering to the said subject a neurologically effective AMPA antagonistic amount of a compound having the formula I

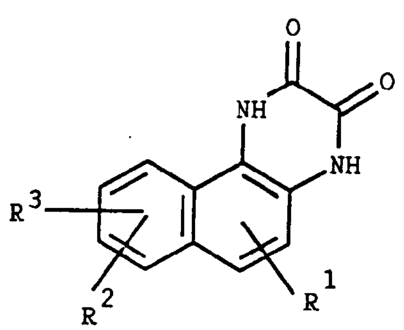

(I)

wherein
$R^1$ is hydrogen, $NO_2$, CN, $CF_3$ or halogen, $R^2$ and $R^3$ independently are hydrogen, CN, $CF_3$, halogen, $C(NOH)C_{1-6}$-alkyl, $COR^4$ or $SO_2R^4$, wherein $R^4$ is $C_{1-6}$-alkyl, optionally substituted with $CONH_2$, or $NR^5R^6$,

wherein $R^5$, $R^6$ independently are hydrogen, $C_{3-6}$-cycloalkyl, $C_{1-6}$-alkyl, optionally substituted with $CONH_2$ or 2-oxo-pyrrolidinyl, or pharmaceutically acceptable salts thereof.

9. A method according to claim 8, wherein the indication is related to cerebral ischemia.

10. A method of preparing a compound according to claim 1, which comprises

a) reacting a compound having the formula II

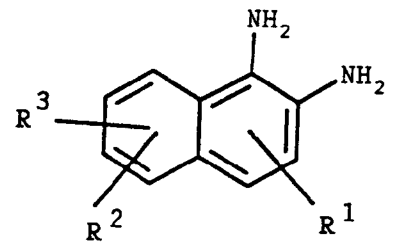

(II)

wherein $R^1$, $R^2$ and $R^3$ have the meanings defined above, with oxalate or a reactive derivative thereof to form a compound of formula I, or

b) refluxing a compound having the formula III

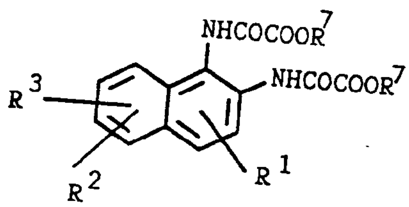

(III)

wherein $R^1$, $R^2$ and $R^3$ have the meanings defined above and $R^7$ is lower alkyl in a mineral acid, to form a compound of formula I, or

c) nitrating a compound having the formula IV

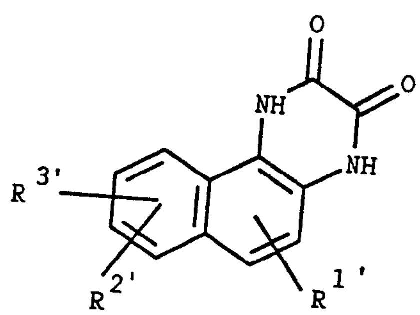

(IV)

wherein at least one of $R^{1'}$, $R^{2'}$ and $R^{3'}$ is hydrogen and the others are as defined for $R^1$, $R^2$ and $R^3$ respectively, to form a compound of formula I, or

d) reducing a compound having the formula V

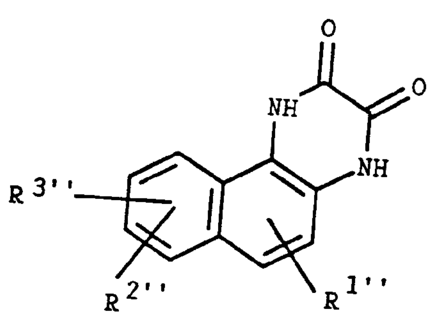

(V)

wherein at least one of $R^{1''}$, $R^{2''}$ and $R^{3''}$ is nitro and the others are as defined for $R^1$, $R^2$ and $R^3$ respectively,

to form a compound of formula I, wherein at least one of $R^1$, $R^2$ and $R^3$ is amino, or

e) reacting a compound having the formula VI

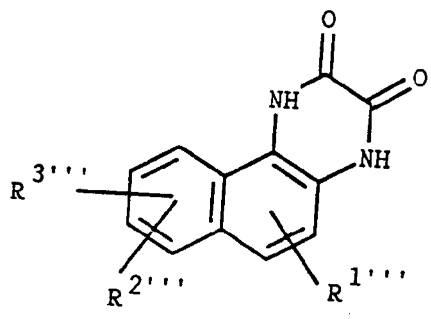

(VI)

wherein at least one of $R^{1'''}$, $R^{2'''}$ and $R^{3'''}$ is $N_2^+$ and the others are as defined for $R^1$, $R^2$ and $R^3$ respectively, with potassium tetracyanonickelate to form a compound of formula I, wherein at least one of $R^1$, $R^2$ and $R^3$ is CN.